# EUROPEAN PATENT SPECIFICATION

(11) **EP 2 658 876 B1**
(45) Date of publication and mention of the grant of the patent: **05.11.2014**
(21) Application number: 11810846.3
(22) Date of filing: 28.12.2011
(51) Int. Cl.: C08B 37/00, C08L 5/08, A61K 31/737

(54) **METHOD FOR THE PREPARATION OF SODIUM CHONDROITIN SULPHATE**
VERFAHREN ZUR HERSTELLUNG VON NATRIUMCHONDROITINSULFAT
PROCÉDÉ DE PRÉPARATION DE CHONDROÏTINE SULFATE DE SODIUM

(30) Priority: 28.12.2010 FR 1061324
(43) Date of publication of application: 06.11.2013
(73) Proprietor: Pierre Fabre Médicament, 92100 Boulogne-Billancourt (FR)
(72) Inventor: CHOMARAT, Nadine, F-81150 Fayssac (FR); GRECH, Paul, F-81600 Gaillac (FR); CUETO, Guillermo, Lujan 1677 (AR); RODRIGUEZ, Edouardo, F-81000 Albi (FR); BOE, Jean-François, F-31520 Ramonville Saint Agne (FR)
(74) Representative: Regimbeau
(86) International application number: PCT/EP2011/074180
(87) International publication number: WO 2012/089777

(56) References cited:
- WO-A1-2009/013550
- FR-A1- 2 756 828
- FR-A1- 2 847 256
- GB-A- 2 098 232
- US-A1- 2003 032 620
- LUO X M ET AL: "CHICKEN KEEL CARTILAGE AS A SOURCE OF CHONDROITIN SULFATE", POULTRY SCIENCE, CHAMPAIGN, IL, US, vol. 81, no. 7, 1 January 2002 (2002-01-01), pages 1086-1089, XP001204549, ISSN: 0032-5791

## Description

The present invention concerns a method for obtaining sodium chondroitin sulphate preparations, notably from cartilage of avian origin.

Chondroitin sulphate is a glycosoaminoglycan found in connective tissue. It is a sulphated polysaccharide comprised of glucuronic acid and N-acetyl galactosamine dimers.

In the tissues, chondroitin sulphate polymers are covalently bound to protein chains to form proteoglycans. Chrondroitin sulphate is well known for its therapeutic effects, notably in the treatment of pain caused by osteoarthritis.

Many publications describe the production of chondroitin sulphate from animal cartilage such as cow, sheep, pig, horse, shark, fish and whale cartilage.

Conventionally, chondroitin sulphate is extracted from cartilage of bovine origin, notably from bovine trachea.

The general principal for extraction of chondroitin sulphate is as follows: the cartilage is subjected to alkaline or acid de-proteinization to remove the glycoproteins with which chondroitin sulphate is associated in the natural state. De-proteinization is then followed by purification of chondroitin sulphate by means of dialysis, separation on ion-exchange resins or precipitation with quaternary ammonium salts of the lysate resulting from the de-proteinization. The chondroitin sulphate obtained in this way is then precipitated using an organic solvent then dried.

Patent FR 2 756 828 concerns a method for the preparation of chondroitin sulphate from avian cartilage. Chondroitin sulphate is obtained by an enzymatic reaction using an alkaline protease of fungal origin, for example Alcalase®, decantation then ultrafiltration. The filtrate then undergoes chemical hydrolysis with hydrochloric acid in order to reduce the molecular weight of chondroitin sulphate. The solution is then neutralised with soda after which chondroitin sulphate is isolated by precipitation and washing in ethanol.

Patent EP 1 560 856 concerns a method for the preparation of chondroitin sulphate from cow, sheep, pig, horse, bird and fish cartilage. Chondroitin sulphate is obtained by an enzyme reaction using a protease followed by precipitation, separation and filtration. The precipitation step is carried out with alkaline earth hydroxide and is followed by a separation step to eliminate the precipitated impurities. Chondroitin sulphate is obtained after the filtration and drying steps.

The purpose of this invention is a new method for obtaining chondroitin sulphate, notably of avian origin. The term "avian origin" in the context of the present invention preferentially means originating from chickens, cocks, turkeys, ducks and geese.

Events relating in particular to bovine spongiform encephalitis, called Mad Cow Disease, led to concerns about the safety of chondroitin sulphate depending on its origin. With the recent appearance of H5N1 virus, or Bird Flu, this problem now also concerns chondroitin sulphate of avian origin.

This invention proposes to remedy this disadvantage. In fact it concerns a chondroitin sulphate preparation presenting increased microbiological and viral safety, in other words free of any germs responsible for diseases contracted by mammalian and bird species.

Moreover, the sodium chondroitin sulphate preparation of the invention presents a more homogeneous sodium chondroitin sulphate population in terms of molecular weight.

In addition, the method according to the present invention makes it possible to obtain chondroitin sulphate in a reproducible manner, whatever the original quality of the cartilage. This method also makes it possible to prepare high-quality chondroitin sulphate with high density, therefore allowing incorporation of a substantial quantity of the product in the desired galenic form.

The sodium chondroitin sulphate preparation according to the method of the invention comprises over 90%, preferably over 95% and even more preferably over 98% sodium chondroitin sulphate (percentage by mass with respect to total mass of the preparation).

The sodium chondroitin sulphate preparation according to the method of the invention preferentially has a polydispersity index less than or equal to 1.47 measured by steric exclusion chromatography SEC-MALLS.

Preferentially said polydispersity index is between 1.22 and 1.47, for example it is 1.37, measured by steric exclusion chromatography SEC-MALLS.

The polydispersity index (calculated from the formula Mw/Mn) makes it possible to estimate polymer dispersion. A lower index indicates that the sodium chondroitin sulphate population in the preparation is less dispersed by molecular mass. This means that the homogeneity of the sodium chondroitin sulphate preparation is of better quality and that the preparation is therefore safer to use.

The sodium chondroitin sulphate preparation according to the method of the invention preferentially has a mean molecular mass by weight (Mw) greater than or equal to 20,000 Daltons, for example greater than or equal to 20,700 Daltons, measured by steric exclusion chromatography SEC-MALLS

Preferentially said mean molecular mass by weight (Mw) is between 20,000 Daltons and 24,000 Daltons, for example between 20,700 Daltons and 23,000 Daltons, for example it is 22,000 Daltons, measured by steric exclusion chromatography SEC-MALLS.

The sodium chondroitin sulphate preparation according to the method of the invention preferentially has a mean molecular mass by number (Mn) greater than or equal to 14,000 Daltons, for example greater than or equal to 14,100 Daltons, measured by steric exclusion chromatography SEC-MALLS.

Preferentially said mean molecular mass by number (Mn) is between 14,000 and 18,000 Daltons, for example between 14,100 Daltons and 18,000 Daltons, for example it is 16,200 Daltons, measured by steric exclusion chromatography SEC-MALLS.

The sodium chondroitin sulphate preparation according to the present invention preferentially has a density between 0.8 and 1, for example a density of 0.9.

This high density is very advantageous because it offers the possibility of incorporating a substantial amount of the product into the desired galenic form, a for example a tablet or capsule.

The sodium chondroitin sulphate preparation according to the method of the invention preferentially presents a peak maximum molecular mass (Mp) greater than or equal to 22,000 Daltons (for example greater than or equal to 22,100 Daltons), measured by steric exclusion chromatography SEC-MALLS.

Preferentially said peak maximum molecular mass (Mp) is between 22,000 and 24,000 Daltons, for example between 22,100 Daltons and 23,300 Daltons, for example it is 22,600 Daltons, measured by steric exclusion chromatography SEC-MALLS.

The sodium chondroitin sulphate preparation according to the method of invention preferentially contains less than 0.09% proteins measured by the Bradford method (percentage by mass compared to total mass of the preparation)

Preferentially, said sodium chondroitin sulphate preparation contains between 0.05% and 0.09% proteins, for example between 0.05 and 0.06% proteins measured by the Bradford method.

The sodium chondroitin sulphate preparation according to the method of the invention preferentially contains less than 2.0% proteins measured by the Lowry method (percentage by mass with respect to total mass of the preparation).

Preferentially, said sodium chondroitin sulphate preparation contains between 1.7% and 2.0%, for example between 1.7 and 1.8% proteins measured by the Lowry method.

Measurement of the quantity of protein in the sample makes it possible to estimate the purity of the sample and therefore its quality (greater safety).

Preferentially, the sodium chondroitin sulphate preparation according to the method of the invention does not contain any or contains quantities that are not detectable by steric exclusion chromatography SEC-MALLS of sodium chondroitin sulphate aggregates and high molecular mass compounds of a size greater than 10⁶ g/mol.

The absence of chondroitin aggregates is advantageous as aggregates are difficult to solubilise and their presence in a pharmaceutical composition therefore makes the active principal less bioavailable.

Preferentially, the preparation according to the present invention presents or includes at least two of the parameters: Mp, Mn, Mw, density, polydispersity index, quantity of proteins, aggregates as defined above.

Even more preferentially, the preparation according to the invention has a polydispersity index as defined above and at least one other of the defined parameters: Mp, Mn, Mw, density, polydispersity index, quantity of proteins, aggregates as defined above.

In another embodiment, the preparation according to the invention has a polydispersity index and includes a quantity of proteins such as that defined above and optionally at least one other of the following parameters: Mp, Mn, Mw, density, aggregates such as defined above.

The present invention concerns a method for the preparation of sodium chondroitin sulphate comprised of three main steps: solubilisation of the animal material by hydrolysis of the cartilage material, securisation and purification of sodium chondroitin sulphate, and precipitation/crystallisation of the product. The method according to the invention makes it possible to obtain a homogeneous and reproducible sodium chondroitin sulphate preparation that can be used without risk.

The method for preparing sodium chondroitin sulphate from cartilage according to the invention comprises or consists of the following steps:
a. Hydrolysis of the cartilage in aqueous medium,
b. Thermal treatment of the hydrolysis between 90 and 100°C for 2 to 10 hours,
c. Separation of the sodium chondroitin sulphate solution from the hydrolysate,
d. Flocculation of the sodium chondroitin sulphate solution and elimination of the flocculate,
e. Purification of sodium chondroitin sulphate in the solution obtained in step d. by ultrafiltration,
f. Treatment of the purified sodium chondroitin sulphate solution obtained in step e. by the addition of hydrogen peroxide at a concentration of 0.02 to 0.2% by mass of hydrogen peroxide with respect to total mass of the purified sodium chondroitin sulphate solution, and
g. Precipitation of the sodium chondroitin sulphate preparation and isolation of the precipitate from the sodium chondroitin sulphate preparation.

In another embodiment of the method for the preparation of sodium chondroitin sulphate from cartilage of avian origin in accordance with the invention, this involves the following steps:
1. solubilisation of the animal material by
   i) proteolytic hydrolysis of this cartilage;
2. securisation and purification by
   ii) thermal treatment;
   iii) separation of chondroitin sulphate;
   iv) flocculation;
   v) purification of the medium at basic pH;
   vi) treatment with bases;
   vii) treatment with hydrogen peroxide;
3. and crystallisation of the product.

Solubilisation of the animal material is carried out by hydrolysis in aqueous medium of the cartilage (step a.). Preferentially, the hydrolysis is enzymatic hydrolysis, even more preferentially proteolytic hydrolysis. The hydrolysis of cartilage makes it possible, in particular, to destructure tissues and solubilise the constituents of cartilage. The amount of water added to the cartilage is dependent on the quantity of cartilage and can be easily established by the man skilled in the art, preferentially it is between 1 and 3 L of water per kg of cartilage.

According to an embodiment of the method of the invention, proteolytic hydrolysis is carried out in the presence of a protease under the optimum temperature, concentration and pH conditions of the enzyme, for example Alcalase, such as Alcalase 3.0T®.

Prior to this hydrolysis step, the cartilage is preferentially ground to make the medium more accessible to the step after hydrolysis.

Securisation according to the invention includes a thermal treatment step of the hydrolysate obtained in step a. (step b.). According to one embodiment of the invention, thermal treatment is carried out between 90 and 100°C, for example 93°C for 2 to 10 hours, preferentially between 3 and 7 hours, even more preferentially at least or around 3 hours. This step has a number of advantages: it makes it possible to inactivate any viruses that may be present, it facilitates the subsequent chondroitin sulphate separation step and it inactivates the enzyme used for cartilage hydrolysis.

Preferentially, the sodium chondroitin sulphate solution separation step (step c.) from the rest of the hydrolysate is carried out by decantation or centrifugation, preferentially by decantation, for example heated decantation. Decantation is a well known method to the man skilled in the art.

The term "separation of chondroitin sulphate" in the context of the present invention means separation of the solid phases (bone residues) from the aqueous phase containing chondroitin sulphate and the oily phase, followed by isolation of the different phases and recovery of the aqueous phase containing chondroitin sulphate.

The aqueous phase containing chondroitin sulphate obtained in step c. then undergoes a flocculation step (step d.). In particular, flocculation is carried out by the addition of a base, notably sodium hydroxide, such that a basic pH greater than 10 is obtained, for example 11 ± 0.5. The flocculation step is carried out at a temperature between 25 and 60°C, for example 50 or 49°C with stirring. The duration of the flocculation step can be easily established experimentally by the man skilled in the art by monitoring the appearance of the flocculate.

The addition of a base leads to the formation of a flocculate, notably one that is protein like. There is precipitation of protein residues and impurities.

The flocculate is then eliminated, for example by filtration or centrifugation, preferentially by filtration notably filtration on diatomaceous earth, and the solution containing chondroitin sulphate filtered in this way is then recovered.

This flocculation, and the flocculate elimination step in particular, facilitate the subsequent steps of the method. In particular, the removal of proteins prevents filter clogging during subsequent filtration steps. Carrying out this step in a basic medium also makes it possible to limit any microbiological growth.

The sodium chondroitin sulphate solution obtained in step d. is then purified (step e.). Preferentially, purification i s carried out by ultrafiltration/diafiltration, notably tangential ultrafiltration with a membrane with a 3 to 30 kDa cut off, for example 10 kDa, depending on the desired chondroitin sulphate molecular mass.

The tangential ultrafiltration process is well known to the man skilled in the art who will easily be able to establish the experimental parameters (including pressure, flow rate) to be applied as a function of the material at his disposal and the supplier's instructions.

This ultrafiltration step makes it possible to eliminate all molecules with a molecular mass below that of the desired chondroitin sulphate and likely to contaminate it, such as hydrolysed proteins, hydrolysed glycoproteins, mineral salts, etc.

According to a particular embodiment of the invention, the method can include a molar mass reduction step for chondroitin sulphate after step e. Advantageously, said molecular weight reduction step is carried out by the addition of hydrochloric acid at a concentration, temperature and duration determined according to the desired final molecular weight. The man skilled in the art can easily establish these parameters by means of routine tests.

In the course of ultrafiltration, the sodium chondroitin sulphate solution is diluted in water. Step e. can therefore be followed by a basic treatment step if the pH of the solution after step e. (or after the potential molecular mass reduction step) is below 10.

This optional step is carried out by addition of a base, notably sodium hydroxide, to the preparation until a pH greater than or equal to 10, preferably greater than or equal to 11, is obtained. Treatment with the base can be extended to one hour with stirring. This step allows the hydrolysis of protein compounds that are to be eliminated to be completed. This treatment can also eliminate amino acid residues which are still bound to the chondroitin sulphate and can limit microbial growth.

The solution obtained in step e. (or after base treatment) is then treated with hydrogen peroxide (step f.). This step is carried out by the addition of hydrogen peroxide, for example 30 V hydrogen peroxide at a final concentration of 0.02 to 0.2% by mass of hydrogen peroxide with respect to total mass of the solution (and/or from 0.1 to 0.5 % v/v, preferably around 0.4%, when using 30 V hydrogen peroxide).

This treatment is carried out at basic pH (for example around 9) at a temperature between 45 and 55°C, for example between 47 and 51°C for a length of time less than 1 hour, for example between 10 minutes and 1 hour, preferably between 10 and 25 minutes, even more preferably between 10 and 20 minutes, for example around 20 minutes.

The thermal treatment step eliminates viruses and significantly reduces microbial populations present in the chondroitin sulphate solution but this does not constitute complete sterilisation. There is therefore still some microbial pollution during the process, with possible growth of micro-organisms not eliminated by heating. Micro-organisms, and notably bacteria, only develop in solution and the powder form of the product does not change. Thus it is necessary to eliminate this disadvantage before the product precipitation process. Different processing solutions have been envisaged for microbial safety: sterilising filtration, thermal treatment and treatment with H₂O₂. Sterilising filtration is not feasible as the purified and concentrated product is too viscous to be filtered.

The inventors have surprisingly found that treatment with hydrogen peroxide guarantees high microbiological quality of the preparations according to the invention, and this with only water and oxygen as the derivative products. Moreover, it is surprisingly found that treatment with hydrogen peroxide under the conditions of the invention does not depolymerise the chondroitin sulphate polymers.

The sodium chondroitin sulphate preparation is then precipitated/crystallised (step g.). Precipitation of the product can be carried out, in particular, by precipitation in ethanol, methanol, acetone or their mixtures, preferentially in ethanol.

In one embodiment of the method, the ethanol precipitation step is carried out by running the chondroitin sulphate solution obtained in step f. in 3 to 10 times its volume of ethanol, preferentially 3 to 5 times, for example 3 times its volume of ethanol, with robust mechanical stirring. In this way the aqueous phase and organic phase are mixed immediately and the precipitate that forms is therefore dispersed.

The precipitate is then isolated. In one embodiment, isolation is carried out by decantation, filtration or centrifugation, preferentially decantation, and elimination of the supernatant.

In one embodiment, the precipitate is then dehydrated by addition of alcohol (for example, 93° ethanol) until an alcohol degree of 80 to 85° is obtained, for example 83° ± 1°, under continuous stirring. The pH of the medium can be maintained at a value between 7.5 and 8.0 by the addition of hydrochloric acid or sodium hydroxide.

The method according to the invention can include, after the precipitation step, a purified chondroitin sulphate drying step in order to obtain a dry powder. In one embodiment of the invention, purified chondroitin sulphate is dried in the oven under vacuum, preferably at a pressure below 150 mBa r, at a temperature greater than 90°C, preferably between 90 and 100°C.

The method according to the invention can include a final conditioning step of the product, possibly preceded by grinding to achieve the desired granulometry.

According to one embodiment of the method according to the invention, the cartilage organs used in this invention are in the fresh or frozen state. Cartilage can also be in a dried or salted form.

The cartilage likely to be used in accordance with the method of the invention preferentially originates from avian species, notably chickens, cocks, turkeys, ducks or geese. The quality of the raw material used varies from very clean cartilage to cartilage contaminated by other tissues.

Preferably, the cartilage is chosen from among wishbones, trachea and joint cartilage.

Another subject-matter of the present invention concerns the preparation of sodium chondroitin sulphate obtained by implementing the method of the invention.

Another subject-matter of the present invention concerns a pharmaceutical composition comprising a sodium chondroitin sulphate preparation according to the invention or obtained by the method according to the invention, and at least one pharmaceutically acceptable excipient.

Said composition according to the invention can notably be in the form of a unit dose for oral administration. Advantageously, each unit dose contains between 250 mg and 1 g of sodium chondroitin sulphate, for example 500 mg.

The oral form is chosen from amongst tablets, capsules, granules, powders, aqueous solutions, emulsions and suspensions.

Another subject-matter of the present invention concerns the sodium chondroitin sulphate preparation according to the invention or obtained by implementing the method according to the invention for use as a medication.

More particularly, the invention concerns the use of said sodium chondroitin sulphate preparation according to the invention for the prevention and/or treatment of osteoarthritis. Advantageously, it is used for the prevention and/or treatment of osteoarthritis of the knee and/or hip.

Preferentially, the treatment of osteoarthritis consists of 1 to 2 g per day of said sodium chondroitin sulphate preparation, for example 2 g, for a patient presenting this disease.

Preferentially, the preparation or pharmaceutical composition according to the invention is used as medication by administration to a patient at a dose of 1 to 2 g per day, for example 2 g.

The description refers to the appended figures wherein:
- Figure 1 represents the minimum logarithmic reduction in the thermal denaturation step of chicken cartilage hydrolysate for the 4 viruses studied;
- Figure 2 represents the distribution of 6 batches according to residual protein content as a function of yield;
- Figure 3 represents the distribution of 6 batches according to chondroitin sulphate content as a function of yield;
- Figure 4 represents analysis by SEC with refractometric detection of 20 batches of avian chondroitin sulphate prepared according to the previous method and according to the method of the invention;
- Figure 5 represents analysis by SEC with determination of molecular weight by MALLS of 20 batches of avian chondroitin sulphate prepared according to the previous method (A) (method in patent application FR 2 756 828 described below) and according to the method of the invention (B).

### EXAMPLE 1: METHOD FOR OBTAINING SODIUM CHONDROITIN SULPHATE

### - Preparation method according to the invention

### Step 1: solubilisation of raw material.

After defrosting the raw material, grinding is carried out. The ground material is mixed with water, at a rate of 2.3 litres per kg of starting material. The medium is heated until a temperature 60°C is achieved evenly throughout the digestion reactor.

The pH of the medium is adjusted to 7.5 - 8.0 by the addition of 30% sodium hydroxide with constant mechanical stirring.

The protease, Alcalase® 3.0T (Subtilisine Carlsberg) is added to the medium at a rate of 7 kg per tonne of cartilage. The enzyme is added in 4 batches of 25% of the total quantity at intervals of 2 hours. The duration of proteolytic hydrolysis is 8 hours minimum, with enzyme additions every 2 hours and pH adjustment to around 7.5 and 7.0 in the course of digestion.

At the end of enzyme digestion, the medium is homogeneous: complete dissolution of cartilage with possibly some bone residues if the starting material contained these.

Step 2: securisation and purification of chondroitin sulphate

The pH of the hydrolysate is adjusted to 5.8 - 6.0 by the addition of 32% hydrochloric acid. The medium is then heated to a minimum temperature of 93°C for at least 3 hours with mechanical stirring. The purpose of this process is to ensure thermal denaturation of the enzyme and inactivation of conventional viruses as well as to promote the separation of fats.

Stirring is stopped and the chondroitin sulphate solution is separated by decantation at a hot temperature greater than 50°C minimum. During cooling, grease decants out to form an upper phase while the undigested residues such as bone fragments fall to the bottom of the reactor. The lower phase consists of the hydrolysate containing chondroitin sulphate. The aqueous phase is titrated and filtered on a press filter. The greasy upper phase is eliminated.

The pH of the hydrolysate is adjusted to 11 ± 0.5 by the addition of 30% w/w sodium hydroxide. The temperature is maintained around 49°C. A protein flocculate forms which is gently decanted. It is eliminated by filtration on diatomaceous earth. The medium is polished on a 1µm pore filter.

Purification of the hydrolysate is carried out by tangential filtration on a 10 kDa membrane.

The process starts with a concentration step at pH 11 and a temperature of 49 ± 2°C. For this step, concentration up to a final volume of 36.5% of the initial hydrolysate volume is carried out (that is a volume concentration factor VCF of 2.75). Next, continuous diafiltration is carried out with a minimum of 4 volumes of water with respect to the volume of initial concentrate in order to eliminate protein residues and mineral salts. This ends with a final over concentration to obtain a chondroitin sulphate concentration in the order of 5 ± 0.5°Brix (which can be likened to a dry extract, in production).

A final concentration is carried out by thermal route with evaporation of water in a LUWA type thin film evaporator (vacuum below 0.3 bar, temperature in the order of 55-75°C, flow rate of 1500 - 2000 litres/hour) to achieve a final concentration in the order 8 to 8.5°Brix.

Basic treatment completes the purification of chondroitin sulphate: 30% sodium hydroxide NaOH sufficient quantity for a final concentration of 0.2N ± 0.05N. The temperature of the medium is adjusted to 35°C ± 2°C. This is left to react for one hour with mechanical stirring, followed by neutralisation by the addition of 32% hydrochloric acid HCl, sufficient quantity for pH 9 ± 0.2.

The purified and concentrated chondroitin sulphate solution is heated to 49 ± 2°C. 0.4% v/v of 30V hydrogen peroxide is added. After stirring for 20 minutes, the solution is neutralised at pH 7.0 ± 0.2 with 32% hydrochloric acid.

### Step 3: recovery of the product in powder form.

Chondroitin sulphate is recovered in solid form by ethanolic precipitation.

The purified chondroitin sulphate solution (1 volume) is run over recycled 93° ethanol (3 volumes with respect to the volume of the aqueous phase) with high mechanical stirring. At the end of the run, stirring is continued for at least 30 minutes before being stopped after a minimum of 8 hours of decantation, the supernatant is titrated and sent to the distiller. 93° ethanol distilled to obtain a final alcohol degree of 83° is added, and possibly 32% hydrochloric acid to achieve a pH of 7.5 to 8.0. This is left to react for around 20 minutes.

The chondroitin sulphate precipitate is recovered by centrifugation. The rotary dryer is supplied by the alcohol medium up to maximum capacity. The supply is stopped to carry out clearing of the medium before discharging the dried cake into containers. The process is repeated until the precipitation reactor is completely emptied. The chondroitin sulphate precipitate can also be recovered by filtration on a stainless steel Buchner filter.

The contents are transferred to the drying zone. The cake is spread over drying trays which are then placed in drying cupboards. The drying parameters are a temperature of 95-100°C, vacuum below 150 mBar and duration of drying greater than 2 hours. Monitoring of drying makes it possible to determine the end of drying. The product is recovered then ground and packaged.

Chondroitin sulphate yield: around 32 kg of chondroitin sulphate per 1000 kg load of frozen avian starting material. The final dry powder has a density greater than 0.8 (between 0.9 and 1).

Depending on the starting material used, the yield can range from 25 to over 40 kg of chondroitin sulphate per load of 1000 kg of avian starting material, without affecting the quality of the final product.

### - Preparation method according to the prior art described in FR 2 756 828:

### Grinding and enzymatic hydrolysis:

3000 kg of frozen chicken wishbones (corresponding to around 1,000,000 chickens originating from health monitored farms) are ground in a food processor type grinder (ACEC) until a homogeneous mixture is obtained.

The grinder is manually fed with loads of 25 kg of frozen wishbones. At the end of grinding of each load, drinking water is supplied to dilute the ground material which can then be transferred into a 12,000 liter reactor by means of a pump. The reactor used is a stainless steel reactor with a turbine type stirring system and external coil for reheating or cooling. It takes around 3 hours to grind the 3000 kg of frozen wishbones.

When the 3000 kg of ground wishbones are placed in the 12,000 litre reactor, the temperature is adjusted to 60°C by circulation of hot water in the coil, after addition of drinking water in a sufficient quantity to obtain a volume of 8000 litres. Stirring at 60 rpm homogenises the ground mixture and water as well as the temperature. Next, to the mixture maintained at 60°C with stirring, 17 kg of alcalase in powder form is added and dispersed by stirring. The pH is checked every 30 minutes and, if necessary, addition of 30% sodium hydroxide in water is carried out in order to maintain pH between 6 and 7.

The hydrolysis process lasts 8 hours.

At the end of 8 hours, the hydrolysate is adjusted to 95°C by circulation of vapour in the coil for 15 minutes in order to carry out thermal denaturation of the alcalase.

Stirring is stopped and the mixture is allowed to cool to room temperature down by circulation of cold water (25°C - 30°C).

### Separation:

### 1. Decantation:

Once the hydrolysis has cooled down, it is left to rest for 8 to 10 hours while maintaining circulation of cold water in the coil. After elimination of the supernatant, the hydrolysate is transferred to a 15,000 litre stainless steel reactor.

### 2. Ultrafiltration

The hydrolysate volume is adjusted to 8,000 litres with drinking water then filtration on Celite® is carried out using 100 kg of Celite, half of which is used to form a "pre-layer" of press filter plates, while the other half is added to the hydrolysate.

The filter is collected in a glassfiber reactor with a capacity of 15,000 liters and equipped with a marine double helix stirring system, coated on the interior with epoxy resins.

Ultrafiltration is carried out in a Millipore apparatus equipped with eighteen membranes of 4.6 ^{m2} each (Helican UF50 made of polyether sulphone per 30 KDa) to concentrate the solution to a volume of 5,000 liters which, after filtration on the press filter in the presence of Celite®, is transferred to a 6,000 liter polyethylene tank. The pH of the filtrate is adjusted to between 6.5 and 7.5 by the addition of 35% hydrochloric acid solution.

### Reduction of molar mass:

The hydrolysate undergoes hydrolysis by 1N hydrochloric acid at 48°C for 5 - 6 hours. It is neutralised by sodium hydroxide then cooled.

Next, sodium hydroxide micropellets are added in a sufficient quantity to obtain a normal solution (40g/L), with the solution stirred at 60 rpm. Solubilisation of sodium hydroxide is verified by measurement of the pH (13.8 to 14). Rapid and intermittent stirring (300 rpm) maintains the homogeneity of the medium for 1 hour while the temperature is maintained at a constant rate of 25-30°C by a graphite exchanger.

The solution is then neutralised (pH 6.7 to 7) by 35% hydrochloric acid (around 300 litres) while maintaining the temperature at 25 - 30°C by means of the graphite exchanger.

### Purification:

The solution obtained in the previous step is transferred into a 20,000 litre stainless steel reactor equipped with an anchored stirrer. Next 15,000 litres of ethanol are added with stirring then sodium chondroitin sulphate is left to precipitate out for around 8 hours. The supernatant is eliminated by aspiration. The precipitate is put in suspension in 20,000 litres of ethanol by stirring at 60 rpm then left to rest for around 8 hours. The supernatant phase is then eliminated. Washing with ethanol is repeated a second time and the supernatant is eliminated again.

The product is filtered through a Büchner type stainless steel filter with a capacity of 300 to 400 kg where any ethanol is eliminated under vacuum of 100 mmHg.

### Drying:

The product obtained is then arranged in an even manner (2 to 3 cm product thickness) on the 28 trays of a stainless steel tray oven in which a vacuum can be maintained and temperature increased.

The temperature is adjusted to 80°C and the vacuum to 200 mmHg. Drying lasts 8 hours at the end of which loss on desiccation is checked (according to Eur. Ph. 2^{nd} Edition V. 6.22 on 1g of the product at 100 - 105°C for 6 hours) which should be between 6.0 and 14.0% before the product is removed from the oven.

The dry product is then packaged in polyethylene bags and cardboard drums. Storage does not exceed 24 hours before the grinding process is started.

### EXAMPLE 2: VIRAL SAFETY

Viral inactivation was checked by doping the cartilage digestion medium with known viral loads. The media were titrated for viruses before and after thermal treatment at 90°C ± 2°C.

The following viruses were tested:
- Virus with RNA and viral envelope:
   ➢ Xenotropic Murine Leukaemia Virus (MuLV), representing non-defective type C retrovirus and having low physico-chemical resistance;
   ➢ Influenza Virus (FLU), constituting a model for avian viruses and having moderate physico-chemical resistance;
- viruses with DNA and without an envelope:
   ➢ Retrovirus 3 (ERO), representing a model for orbivirus, rotovirus and blue tongue bovine virus and having moderate to high physico-chemical resistance;
   ➢ Porcine Parvovirus (PPV), representing a model for human parvovirus B19 and having high physico-chemical resistance.

### Results:

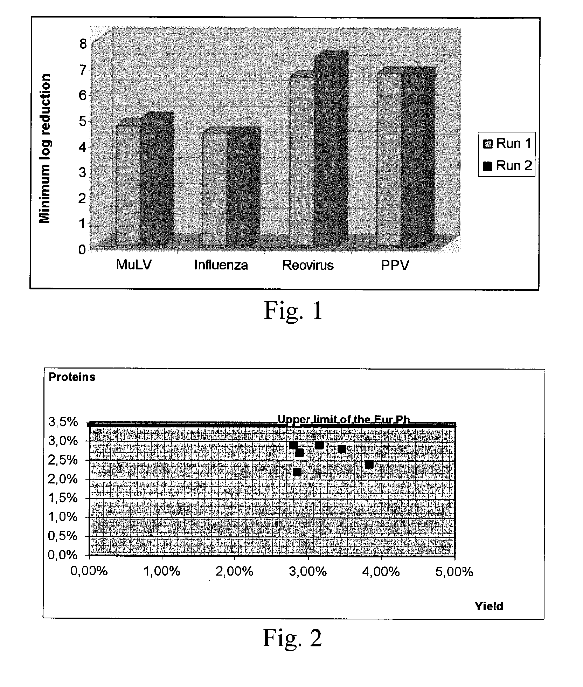

Figure 1 indicates for each virus tested the minimum logarithmic reduction in the thermal denaturation step of the chicken cartilage hydrolysate.

A minimal logarithmic reduction of 4 is provided assured for all viruses, and even > 6 for Reovirus and PPV, by thermal treatment for 3 hours at a temperature of 90°C ± 2°C.

### EXAMPLE 3: ROBUSTNESS AND REPRODUCIBILITY OF THE METHOD

The cartilage starting materials can be contaminated by neighbouring tissues such as meat, fat, bone fragments, etc.

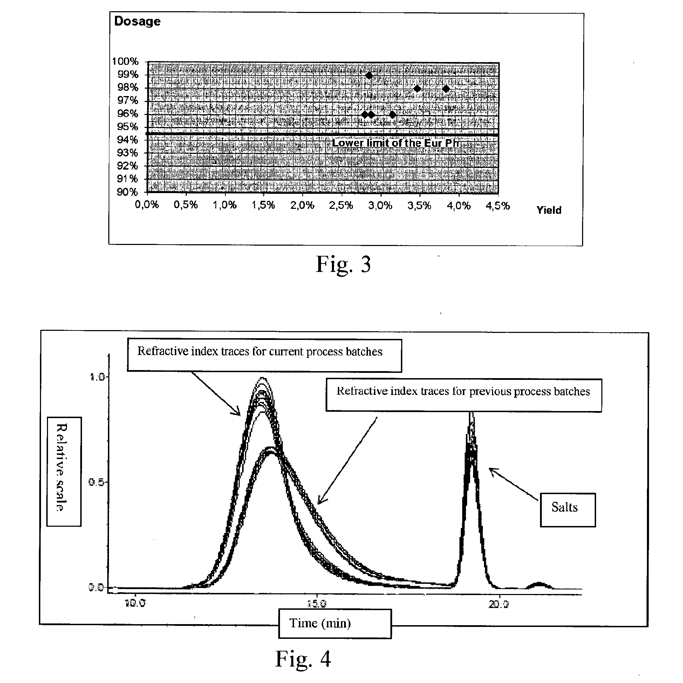

The distribution of 6 production batches was studied according to the two main criteria of product purity, content in residual proteins and chondroitin sulphate assay as a function of yield (Figure 2 and Figure 3).

### Results:

The study of 6 production batches shows that the quality of the chondroitin sulphate produced is constant: chondroitin sulphate titre between 96 and 99%, residual protein content between 2.2 and 2.9%, content in related substances below 2% (Table 1).

**Table 1**

| Batch | Yield (%) | CS Titer (%w/w dry) | Proteins (%w/w dry) | Related substances <2% (Electrophoresis) | Loss on desiccation (%) | Ethanol (%) | pH |
|---|---|---|---|---|---|---|---|
| 1 | 3.46 | 98 | 2.8 | Conforms | 9.9 | C.3 | 6.3 |
| 2 | 3.83 | 98 | 2.4 | Conforms | 9.1 | C.3 | 6.6 |
| 3 | 2.85 | 99 | 2.2 | Conforms | 9.4 | C.3 | 6.7 |
| 4 | 2.80 | 96 | 2.9 | Conforms | 6.4 | C.3 | 6.1 |
| 5 | 3.15 | 96 | 2.9 | Conforms | 74 | C.3 | 6.4 |
| 6 | 2.88 | 96 | 2.7 | Conforms | 8.5 | C.5 | 6.5 |

The other items (appearance, solubility, sodium test, chloride content, infra-red spectrum, heavy metal content and microbiology) are also constant and conform to the specifications.

There is no direct link between the quality of batches produced and the yield obtained; this shows the robustness of the method, capable of using different qualities of starting materials whilst always obtaining a product of constant quality.

Whatever the quality of starting materials, the sodium chondroitin sulphate obtained always complies with European Pharmacopeia specifications.

The presence of these nearby tissues has no negative impact on the quality of the product obtained.

### EXAMPLE 4: PHARMACEUTICAL COMPOSITION

The chondroitin sulphate used is obtained according to the method described in example 1.

| | |
|---|---|
| Sodium chondroitin sulphate | 500 mg |
| Talc | qs |
| Gelatin | qs |
| Titanium dioxide | qs |
| Indigotin | qs |

In examples 5 to 7, the term "previous method" refers to the method described in patent FR 2 756 828.

### EXAMPLE 5: STUDY OF THE DISTRIBUTION OF MOLECULAR MASS BY SEC AND RI/MALLS DETECTION

### Assessment methods

The system consists of an Agilent 1200 series chromatography system with a DAD detector (G1315B). The specific detectors are the MALLS DAWN WOS (Wyatt Technology) laser light multi-angular diffusion system and a differential refractometer (Waters 2414). Bovine serum albumin (BSA), a monodispersed protein with a molecular mass of 66400 Da (a dn/dc value of 0.185, Perbio), is used to normalise the DAWN EOS detector. It is calibrated using a Pullulan reference standard with a molecular mass of 22,800 Da (dn/dc value of 0.145, Polymer Laboratories). Data were analysed with ASTRA software (Wyatt Technology), using a dn/dc value of 0.141 for chondroitin sulphate (as defined in the previous report) and Zimm adjustments of the first order.

BSA is put in suspension in the mobile phase (125 mM Na₂SO₄, 0,03% NaN₃) at a concentration of 1 mg/mL and filtered through a 0.02 µ filter. Pullulan and the chondroitin sulphate batches are put in suspension in the mobile phase at a concentration of 5 mg/ml, incubated overnight with stirring at room temperature and filtered through 0.45 µm filters prior to injection of 100 µL at a flow rate of 0.5 ml/min

### Results

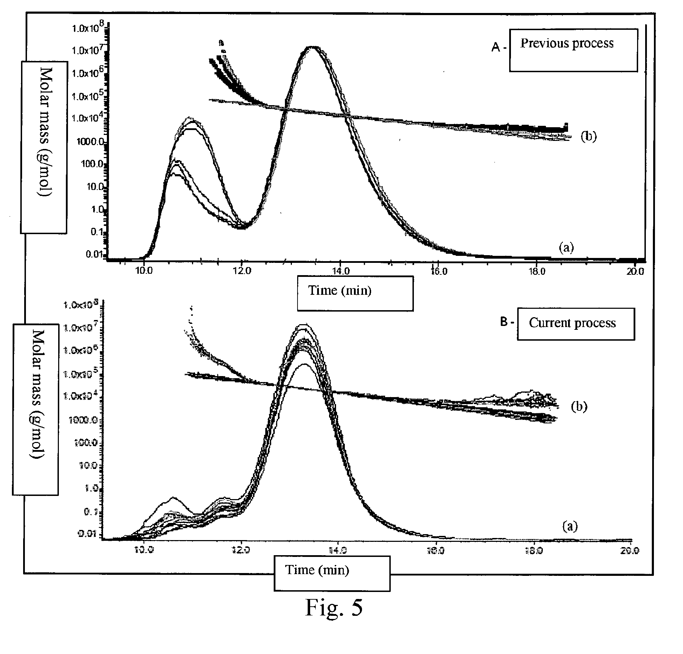

The chromatography profiles of the 20 batches of chondroitin according to the previous method and the method of the invention are obtained by size exclusion chromatography (SEC) with refractometric detection (RI, Figure 4) and determination of molecular mass by MALLS (Figure 5). The mean masses and polydispersion results are presented in Table 2.

Comparison of the methods by SEC analysis with refractometric detection is presented in Figure 4.

The graphs for chondroitin sulphate refraction indices (Figure 4) present an eluent peak at approximately 13.5 minutes for the two methods, corresponding to the distribution of chondroitin molecular mass. The salts are eluted subsequently as illustrated by the peak at approximately 19 minutes and plus.

Differences in the shapes of the peaks are nonetheless observed. In fact batches based on the method of the invention present a homogeneous profile with a more Gaussian population compared to batches using the previous method which have less symmetrical peaks, particular for low molecular masses.

The laser light diffusion plots and mean molecular mass values established by SEC with MALLS detection are presented in Figure 5.

The lines are plots of the diffusion of laser light for a detector at 90° (a) and mean molecular mass values calculated for each time interval with linear adjustment (b).

As observed for the RI signal, the MALLS profile (eluent peak at approximately 13.5 minutes) for avian chondroitin sulphate batches obtained with the method of the invention are more homogeneous.

Moreover, populations with high molecular masses are observed for an elution time between 10 and 12 minutes. These aggregates are more common in batches obtained according to the previous method as can be seen from Figure 5-A.

Whatever the method, aggregates present molecular masses of over 1 x 10⁵ g/mol (see plot b on figure 5) but in very low quantities, as illustrated in Figure 4 where no RI signal is detected between 10 and 12 minutes.

Analysis of the mean molecular mass and polydispersion results are presented in Table 2.

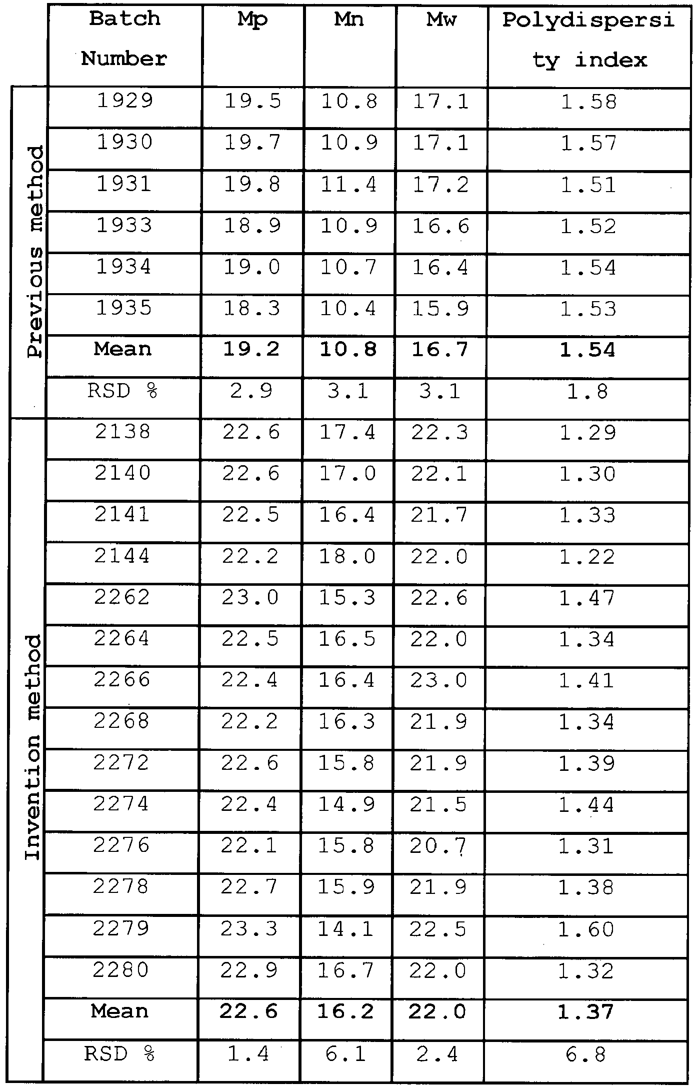

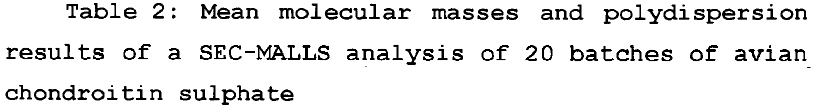

with the molecular mass at the peak maximum (Mp, kDa), mean molecular mass of different chains in numbers (Mn, kDa) or in weight (Mw, kDa) and polydispersity index (Mw/Mn) (RSD: relative standard deviation)

It should be noted that the polydispersity index of 1.60 for batch 2279 corresponds to an aberrant value.

The values are homogeneous for batches produced according to each method, with a relative standard deviation no greater than 7%.

The molecular mass at the peak maximum of the previous method is different from that of the method of the invention with values of 19.2 and 22.6 kDa respectively. This difference is also observed on the chromatograms presented in Figure 5 with eluant peaks at ~ 13.5 minutes (previous method) and ~ 13.2 minutes (method of the invention).

The values for the mean molecular masses by number (Mn) and by weight (Mw) are greater for batches of the method of the invention compared to batches of the previous method.

The polydispersity index of 1.37 for batches produced by the invention method is lower than that for batches produced by the previous method with a value of 1.54, indicating better population homogeneity for the invention batches.

To summarise, compared to the previous method, the method of the invention leads to more homogeneous distribution of molecular mass of the avian chondroitin sulphate population, with a slightly higher molecular masses and fewer large aggregates.

### EXAMPLE 6: QUALITATIVE AND QUANTITATIVE ANALYSIS OF PROTEINS

### Assessment method

### Total protein assay

### - Bradford method

The total proteins assay is carried out in accordance with the European Pharmacopoeia, section 2.5.33, method 3, which is commonly referred to as the Bradford method. This method is based on the shift in absorption from 470 nm to 595 nm observed when Coomassie Blue binds to the protein.

Seven standard bovine serum albumin solutions were prepared from 25 to 1,500 µg/mL of water. Relative to the samples, two preparations per batch of chondroitin sulphate in suspension in water at 100 mg/mL are made up. The samples, standard solutions and control (water) are incubated with the Coomassie reagent for 10 minutes at room temperature. The absorbance of the sample and the standard solutions is determined at 595 nm using the control as the compensation liquid.

### Lowry method

The total proteins assay is carried out according to the European Pharmacopoeia, section 2.5.33, method 2. This method is based on the reduction of the protein in the mixed phosphomolybdotungsten acid chromogen in the phosphomolybdotungsten reagent which results in an absorbance maximum at 750 nm.

Five standard bovine serum albumin solutions are prepared in 0.1 N NaOH, from 25 to 1000 µg/mL. Relative to the samples, two preparations per batch of chondroitin sulphate are put in suspension in water at 50 mg/mL and diluted in 0.1 N NaOH to 10 mg/mL. The samples, standard solutions and control (0.1 N NaOH) are incubated with Lowry reagent for 10 minutes at room temperature. Further reagent is added before incubation for 30 minutes at room temperature. The absorbances of the sample and standard solution are determined at 750 nm using the control as the compensation liquid.

### SDS electrophoresis on polyacrylamide gel

Chondroitin sulphate is put in suspension in water at 100 mg/mL then stirred by rotary stirrer for 30 minutes. The proteins are extracted with methanol/chloroform/water (4/1/3 v/v) and centrifuged at 10,000 g for 10 minutes. The upper and lower phases are carefully withdrawn avoiding any contact with the protein interphase. A second protein extraction is carried out as described above up to the point of the protein interface. The proteins are precipitated with methanol and centrifuged at 14,000 g for 3 minutes. The deposits are dried then re-suspended in 40 µL of load buffer (Bio-Rad 161-0791). The samples are heated for 3 minutes at 95 °C then centrifuged for 2 minutes at 14,000 g.

Ten microlitres (10 µL) of supernatants from the sample and molecular mass marker (LMW, GE, 17-0446-01) are loaded onto Criterion XT Bis-Tris 12% gel (Bio-Rad 345-0118). Gel electrophoresis is carried out using a Criterion cell (Bio-Rad, Hercules, CA) at room temperature and constant voltage of 200 V. The gels are stained with Coomassie Blue R-250, captured with the aid of a photo apparatus (GBOX-Chemi-XT16) and analysed with SYNGENE software.

### Results

### Quantitative analysis by total proteins assays

Whatever the method used, the calibration curve is obtained by graphic representation of the absorbance of standard solutions compared to protein concentrations. The protein concentration is determined in the sample solutions from the calibration and absorbance graph of the sample solution.

The quantity of total proteins is evaluated using two methods, the Bradford method and the Lowry method whose principles differ in terms of interaction with amino acids and peptide size. In fact, the Bradford method mainly presents a reaction with the arginine residues within a protein (without binding to free amino acids) and the Lowry method only allows interaction with the four peptide bonds of tyrosine and tryptophan. Thus the main difference resides in the size of peptides and proteins subjected to tests, with recognition of a higher molecular weight protein for the Bradford method compared to the Lowry method.

The total protein quantities are summarised in Table 3.

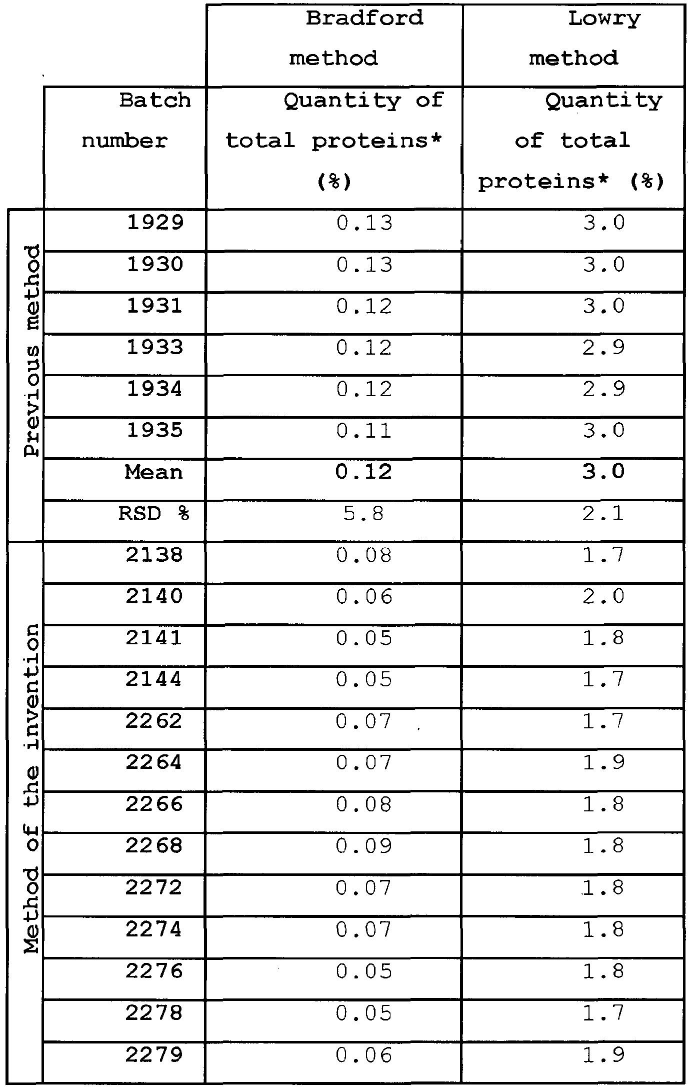

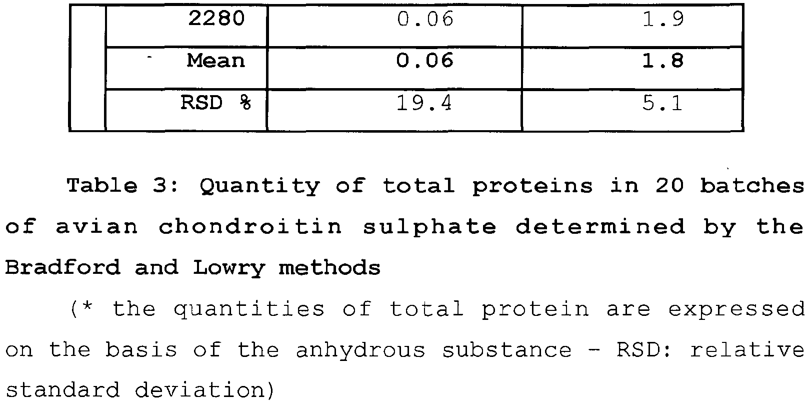

The total protein quantities are less than 0.13% with the Bradford method and less than 3.0% with the Lowry method, both estimated on the basis of the anhydrous substance. The differences between these methods can be correlated to the structure of proteins essentially present in the form of small peptides in the chondroitin sulphate batches.

Moreover, the results obtained for batches using the previous method present values that are approximately two times greater than the values for batches obtained using the method of the invention.

### Qualitative analysis by SDS-PAGE

Electrophoregrams were made up. The electrophoregrams present two types of protein profile as a function of the batches tested. In fact batches produced using the previous method have a more bands (with a molecular mass below 70 kDa) than batches obtained by the method of the invention which only present two diffuse bands (close to 20 and 45 kDa).

### EXAMPLE 7: QUALITATIVE AND QUANTITATIVE DNA STUDIES

### Assessment method

### DNA assay by the diphenylamine method

The assays carried out according to procedure code STANDCOC-36CO0201 with two minor modifications (30 mg instead of 25 mg of chondroitin sulphate and the calibration curve is obtained using an additional point at a low concentration (approximately 0,027 mg of DNA/mL)). In brief, DNA is hydrolysed by trichloroacetic acid. The interaction between free deoxyribose and the diphenylamine reagent is seen by absorbance at 600 nm. Quantification is carried out using a calf thymus DNA calibration curve.

### Extraction of DNA and electrophoresis analysis

Chondroitin sulphate is put in suspension in digestion buffer (60 mM sodium acetate, 50mM Tris-HCl pH 8.0) at 100 mg/mL and incubated for 4 hours with 0.16 units of chondroitinase ABC from *Proteus vulgaris,* Sigma C3667. DNA is extracted with phenol/chloroform/isoamyl alcohol (25/24/1 v/v), precipitated with a volume of 1/10° of 3 M sodium acetate and ethanol at 100 %. The deposit, rehydrated with 70% ethanol, is dissolved in 10 mM tris-HCl, 1 mM EDTA buffer solution, pH 8.0. Agarose gel at 0.8% is used to visualise the DNA extract.

Migration is carried out in 89 mM tris/ 89 mM boric acid/ 2mM EDTA buffer pH 8.0, with constant voltage of 130 V for 2 hours. Three-and-α-half microlitres (3.5 mL) of low range I DNA molecular mass marker (Interchim 467490) are loaded onto the two sides of the gel. Ten microlitres (10 µL) of sample (corresponding to 5 mg of the equivalent medication) are mixed with 2 µL of load buffer 6x and the mixture then placed in each well. After migration, the gel is incubated for 10 minutes in ethidium bromide solution at 1 µg/mL and DNA is visualised under long wave UV light.

### Results

### Quantitative analysis of DNA by the diphenylamine method

The quantities of DNA are summarised in Table 4

**Table 4: quantity of DNA in 20 batches of avian chondroitin sulphate**

| Previous method | | Method of the invention | |
|---|---|---|---|
| Batch number | % DNA (w/w) | Batch number | % DNA (w/w) |
| **1929** | 1.24 | **2138** | 1.26 |
| **1930** | 1.20 | **2140** | 1.18 |
| **1931** | 1.27 | **2141** | 1.28 |
| **1933** | 1.53 | **2144** | 0.95 |
| **1934** | 1.59 | **2262** | 1.30 |
| **1935** | 1.62 | **2264** | 1.14 |
| | | **2266** | 1.17 |
| | | **2268** | 0.91 |
| | | **2272** | 1.19 |
| | | **2274** | 1.33 |
| | | **2276** | 1.08 |
| | | **2278** | 1.33 |
| | | **2279** | 1.38 |
| | | **2280** | 1.42 |
| **Mean** | **1.41** | **Mean** | **1.21** |
| **RSD** % | 13.5 | **RSD**% | 12.5 |

| | | | |
|---|---|---|---|
| (RSD: relative standard deviation) | | | |

Whatever the method used, there is no significant difference between the batches. The quantity of DNA is between 0.9 and 1.6% (w/w) of the medication chondroitin sulphate.

Qualitative analysis of DNA by phenyl/chloroform extraction

Electrophoregrams were made up. No significant difference between the previous method and the method of the invention is found. A weak band of 1,400 base pairs is observed in the majority of batches. Moreover, and independently of the batches tested, extracted DNA is broken down as revealed by the trails of molecular masses below 700 base pairs, corresponding to approximately 230,000 daltons of monoquaternary DNA.

### CONCLUSION

The characterisation of avian chondroitin sulphate by comparing the old production method with the production method of the invention is approached in terms of molecular distribution, and protein and DNA analysis.

Molecular mass distribution determined by SEC-MALLS is more homogeneous with a slightly higher molecular mass and fewer large aggregates in batches produced by the method of the invention compared to batches produced by the previous method.

Protein analysis is carried out both quantitatively and qualitatively.

The quantity of total proteins in batches produced by the previous method and determined by the Bradford and Lowry methods have values that are approximately two times higher than those of batches produced by the method of the invention. Moreover, significant differences are observed depending on the method used (up to 0.13% and 3.0% for the Bradford and Lowry methods respectively). Due to the principles of these methods, this difference can be correlated to the structure of proteins essentially present in the form of small peptides in batches of chondroitin sulphate.

Qualitative analysis of the extracted proteins reveals two profiles on the electrophoregrams as a function of the batch tested. In fact batches produced by the previous method present a larger number of bands (with a molecular mass of less than 70 kDa) than batches produced by the method of the invention which only have two diffuse bands (close to 20 and 45 kDa).

DNA analysis is approached in terms of quantification by the diphenylamine method and characterisation by electrophoresis on agarose gel after phenol/chloroform extraction. There is no significant difference between the batches tested and the method used, with very low quantities of DNA (0.9 and 1.6% w/w) present in degraded forms (molecular weights below 700 base pairs).

## Claims

1. A method for the preparation of sodium chondroitin sulphate from cartilage comprising the following steps:
a. Hydrolysis of the cartilage in aqueous medium,
b. Thermal treatment of the hydrolysis between 90 and 100°C for 2 to 10 hours,
c. Separation of the sodium chondroitin sulphate solution from the hydrolysate,
d. Flocculation of the sodium chondroitin sulphate solution and elimination of the flocculate,
e. Purification of sodium chondroitin sulphate in the solution obtained in step d. by ultrafiltration,
f. Treatment of the purified sodium chondroitin sulphate solution obtained in step e. by the addition of hydrogen peroxide at a concentration of 0.02 to 0.2% by mass of hydrogen peroxide with respect to total mass of the purified sodium chondroitin sulphate solution, and
g. Precipitation of the sodium chondroitin sulphate preparation and isolation of the precipitate from the sodium chondroitin sulphate preparation.

2. Method according to claim 1, **characterised in that** the flocculation step is carried out by the addition of a base such that a pH of above 10 is achieved.

3. Method according to claim 1 or 2, **characterised in that** the chondroitin sulphate precipitation step is carried out by means of ethanolic precipitation.

## Patentansprüche

1. Verfahren zur Herstellung von Natriumchondroitinsulfat aus Knorpelgewebe, umfassend die folgenden Schritte:
a. Hydrolyse des Knorpelgewebes in wässrigem Medium,
b. Wärmebehandlung der Hydrolyse zwischen 90 und 100 °C für 2 bis 10 Stunden,
c. Trennung der Natriumchondroitinsulfatlösung aus dem Hydrolysat,
d. Flokkulation der Natriumchondroitinsulfatlösung und Entfernung des Flokkulats,
e. Aufreinigung des Natriumchondroitinsulfats in der in Schritt d. erhaltenen Lösung durch Ultrafiltration,
f. Behandlung der in Schritt e. erhaltenen gereinigten Natriumchondroitinsulfatlösung durch Zugabe von Wasserstoffperoxid in einer Konzentration von 0,02 bis 0,2 Massen-% Wasserstoffperoxid, bezogen auf die Gesamtmasse der gereinigten Natriumchondroitinsulfatlösung, und
g. Ausfällung der Natriumchondroitinsulfatpräparation und Isolierung des Präzipitats aus der Natriumchondroitinsulfatpräparation.

2. Verfahren gemäß Anspruch 1, **dadurch gekennzeichnet, dass** der Flokkulationsschritt durch Zugabe einer Base, derart, dass ein pH-Wert über 10 erreicht wird, ausgeführt wird.

3. Verfahren gemäß Anspruch 1 oder 2, **dadurch gekennzeichnet, dass** der Chondroitinsulfat-Ausfällungsschritt mittels Ethanolpräzipitation ausgeführt wird.

## Revendications

1. Méthode de préparation de chondroïtine sulfate de sodium à partir de cartilage, comprenant les étapes suivantes :
a. l'hydrolyse du cartilage dans un milieu aqueux,
b. le traitement thermique de l'hydrolyse entre 90°C et 100°C pendant 2 à 10 heures,
c. la séparation de la solution de chondroïtine sulfate de sodium de l'hydrolysat,
d. la floculation de la solution de chondroïtine sulfate de sodium et l'élimination du floculat,
e. la purification de la chondroïtine sulfate de sodium dans la solution obtenue dans l'étape d par ultrafiltration,
f. le traitement de la solution de chondroïtine sulfate de sodium purifiée obtenue dans l'étape e par l'ajout de peroxyde d'hydrogène à une concentration de 0,02 % à 0,2% en masse de peroxyde d'hydrogène par rapport à la masse totale de la solution de chondroïtine sulfate de sodium purifiée, et
g. la précipitation de la préparation de chondroïtine sulfate de sodium et l'isolement du précipité à partir de la préparation de chondroïtine sulfate de sodium.

2. Méthode selon la revendication 1, **caractérisée en ce que** l'étape de floculation est réalisée par l'ajout d'une base de façon qu'un pH supérieur à 10 soit atteint.

3. Méthode selon la revendication 1 ou 2, **caractérisée en ce que** l'étape de précipitation du chondroïtine sulfate est réalisée au moyen d'une précipitation éthanolique.
